Europäisches Patentamt

European Patent Office

Office européen des brevets

(11) Publication number: **0 153 871**
**A2**

(12) # EUROPEAN PATENT APPLICATION

(21) Application number: **85301388.6**

(22) Date of filing: **28.02.85**

(51) Int. Cl.⁴: **A 61 K 39/00**
**C 12 N 5/00, C 12 N 15/00**
**A 61 K 39/29**
**//A61K39/39**

(30) Priority: **01.03.84 US 585145**

(43) Date of publication of application:
**04.09.85 Bulletin 85/36**

(84) Designated Contracting States:
**AT BE CH DE FR GB IT LI LU NL SE**

(71) Applicant: **CENTOCOR, INC.**
**244 Great Valley Parkway**
**Malvern, PA 19355(US)**

(72) Inventor: **Chang, Tse Wen**
**1504 Sugartown Road**
**Paoli Pennsylvania 19301(US)**

(72) Inventor: **Celis, Esteban**
**346 Wrights Lane**
**Exton Pennsylvania 19341(US)**

(74) Representative: **Holdcroft, James Gerald, Dr. et al,**
**Graham Watt & Co. Riverhead**
**Sevenoaks Kent TN13 2BN(GB)**

(54) Antibody enhancement of immunogenicity of antigen.

(57) Methods and compositions for enhancing an immune response of T lymphocytes to antigen are described. Complexes of antigen and antibody specific therefor, formed with a molar excess of antibody, augment proliferative and effector function response fo antigen-specific T lymphocytes *in vitro*. Monoclonal antibody of the $IgG_1$ or $IgG_{2a}$ subclass exhibit the greatest enhancing capability. Antigen-antibody complexes can be used to potentiate *in vitro* activation and clonal expansion of antigen-specific T lymphocytes. Further, complexes of antigen and monoclonal $IgG_1$ or $IgG_{2a}$ antibody specific for the antigen may be used to formulate more efficient vaccine for vaccination *in vivo*.

EP 0 153 871 A2

- 1 -

ANTIBODY ENHANCEMENT OF IMMUNOGENICITY OF ANTIGEN

Description

Technical Field

This invention is in the fields of cell biology and immunology. The invention pertains to methods of enhancing the immune response of lymphocytes to antigens and to methods of immunization in vitro and in vivo and to immunogen compositions.

Background of the Invention

The development of immune response involves intricate interactions between different cell types and immune factors. In general, there are two broad classes of immune response: (1) humoral antibody responses involving antibody production and (2) cell-mediated immune responses involving the production of specialized cells with a variety of functions. The two major classes of immune responses are mediated by two different classes of lymphocytes. T lymphocytes (or T cells) which mature in the thymus are responsible for cell mediated immunity; B cells, which produce antibodies, are the principals in humoral antibody immunity.

T and B cells become morphologically distinguishable only after they have been stimulated by antigen. When B cells are stimulated by antigens, they differentiate into antibody secreting plasma cells. Usually a B lymphocyte cannot be induced to make antibody without help from a T cell. This T cell cooperation requires a simultaneous T cell response to the same antigen.

When T cells are stimulated by antigen, they mature into effector cells that are responsible for the various cell-mediated immune reactions. There are at least four important functional subtypes of effector T cells; helper cells, which interact with B cells to amplify production of antibody and aid other T cells in cell-mediated immune response; suppressor cells, which inhibit the responses of B and T lymphocytes; cytotoxic T cells, which carry out the direct cell-killing function of T cells; and a unique subtype of T cells which mediate delayed type hypersensitivity to some antigens. Because both helper and suppressor T cells act as regulators of the immune response, they are referred to as regulatory T cells. Some helper T cells respond to antigen by secreting substances called lymphokines which stimulate proliferation of other lymphocytes and helper T cells themselves.

An important feature of a T lymphocyte is its unique surface receptor which enables it to recognize a particular antigen, actually a particular antigenic determinant (epitope). A T cell responds only to one antigenic determinant because it exhibits only one kind of surface receptor. This special correspondence provides exquisite specificity.

T cells manifest their response to antigen by developing clones. When a T cell encounters an antigen recognized by its surface receptor, it transforms into a lymphoblast. The lymphoblast then goes on to divide sequentially and exponentially giving rise to a population of T cells with identical specificity, i.e., a clone. The process is known as clonal selection and expansion.

Most T cells respond to an antigen only if the antigen is on the surface of a cell. For example, helper T cells respond to antigen attached to the surface of antigen-presenting cells which in many cases are macrophages, large, highly phagocytic mononuclear cells. Moreover, the antigen must be presented in association with certain glycoprotein molecules also on the surface of the presenting cell. These surface molecules are encoded by genes of the major histocompatibility complex, the portion of the genome which encodes transplantation antigens. Helper T cells triggered by properly presented antigen differentiate, proliferate and secrete factors and influence the production of immunological effector molecules (e.g. antibodies) and effector cells (e.g. cytotoxic T cells).

In order to study the immune response of T cells at the cellular level, immunologists have isolated populations of T cells specific for a particular antigen. For example, in recent years, lines and clones of human T lymphocytes have been established which demonstrate specific reactivity to allogeneic cells, tetanus toxoid, purified protein derivative (PPD) from tuberculin, keyhole limpet hemocyanin and to viruses such as influenza and herpes. Some of these T cell clones are reactive

-4-

with cell surface alloantigens and display cytotoxicity upon their respective target cells; others are reactive with soluble antigens and promote antibody production by B lymphocytes and fall into the T helper subtype.

Immunologists have sought methods of enhancing the immune response to antigens in order to reduce the amount of antigen required for immunization and to potentiate the immunogenicity of weakly antigenic substances. One way of obtaining a stronger immune response to some antigens is to complex the antigen with specific immunoglobulin and immunize with the immune complex. For virus antigens complexed to polyclonal antibody, Houston and co-workers recognized at least two critical factors upon which such a procedure depends. First, it is necessary for the antigen-antibody complexes to be formed at equivalent proportions of antigen and antibody because complexes formed in excess of either antibody or antigen do not yield enhancement. Second, the antibody used to form the complex must be homologous with the species immunized. Houston, N.E. et al., (1977) "Inactivated Venezuelan Equine Encephalomyelitis Virus Vaccine Complexed with Specific Antibody: Enhanced Primary Immune Response and Altered Pattern of Antibody Class Elicited" J. Infect. Dis. 135: 600-610.

The first criterion, that of antigen-antibody complexes formed with equivalent proportions of antigen and antibody, only applies to particulate antigens such as viruses. Stoner and Terres reported that for the soluble antigen tetanus toxoid, complexes formed in antigen excess elicited equal or greater enhancement than did complexes formed at equivalence. Stoner, R.D. and Terres, G. (1963) "Enhanced Antitoxin Responses in Irradiated Mice Elicited by Complexes of Tetanus Toxoid and Specific Antibody" J. Immunol. 91: 761-770.

The requirement of homologous polyclonal antibody, however, apparently applies to soluble as well as particulate antigen because complexes of tetanus toxoid and heterologous antibody cause depression of primary, humoral response. Stoner, R.D. et al. (1975) "Early and Enhanced Antitoxin Responses Elicited with Complexes of Tetanus Toxoid and Specific Mouse and Human Antibodies" J. Infect. Dis. 131: 230-238.

As with the majority of infectious diseases, resistance to hepatitis B virus (HBV) is largely mediated by protective antibodies and immune effector cells. Individuals who recover from an acute HBV infection develop immunity to the virus by producing antibodies to the viral envelope molecule, hepatitis B surface antigen (HBsAg). In fact, individuals with a high risk of HBV infection have been successfully immunized against HBV with an inactivated, purified preparation of HBsAg. Most of the vaccine recipients develop high titers of antibodies to HBsAg (anti-HBs) and the incidence of hepatitis B is decreased markedly among the vaccinees.

-6-

## Disclosure of the Invention

This invention pertains to methods of potentiating immunogenic response to antigens and to immunogen compositions useful in such methods. The method and compositions of the invention are founded upon the discovery that antigen-antibody complexes enhance antigen-induced clonal expansion of T lymphocytes and T lymphocyte effector function. Accordingly, antigen-antibody complexes can be used to augment antigen-specific T cell response to antigens. More potent immunogen and vaccine compositions can be formulated by employing complexes of antigen and antibody specific therefor.

Several human T lymphocyte clones specific for HBsAg were developed from peripheral blood mononuclear cells of recipients of hepatitis B vaccine. The cells proliferate upon incubation with HBsAg in vitro. Purified human polyclonal antibody specific for HBsAg (anti-HBs), but not non-specific antibody, augments the antigen-induced proliferative response. For example, a maximum proliferative response to HBsAg can be achieved at a concentration of HBsAg complexed with anti-HBs ten to one hundred times lower than the concentration of HBsAg required in the absence of anti-HBs.

Monoclonal anti-HBs also enhances HBsAg-induced proliferation of the human T cell clones. In general, monoclonal anti-HBs of the IgG class produce greater enhancement than monoclonal IgM anti-HBs or polyclonal anti-HBs. The monoclonal antibody may be a heterologous antibody. For instance, murine monoclonal anti-HBs enhances the immune response of human T cells to HBs.

These results indicate that antigens complexed to antibody, particularly $IgG_1$ and $IgG_{2a}$ monoclonal antibody, evoke a stronger immune response in T lymphocytes. Antigen-antibody complexes can be used to enhance activation and clonal expansion of T lymphocytes in vitro. Further, monoclonal antibody may be used to produce more efficient vaccines for vaccination in vivo.

Brief Description of the Drawings

Figure 1 illustrates the enhancement of antigen-induced proliferation of HBsAg-specific T cell clones by HBsAg-anti-HBs complexes.

Figure 2 illustrates that anti-tetanus toxoid antibody does not enhance the proliferative response of HBsAg-specific T cell clones to HBsAg.

Figure 3 illustrates the enhancement of HBsAg-induced proliferation of the HBC-6 T cell clone by mouse monoclonal anti-HBs.

Figure 4 illustrates the effect of mouse monoclonal anti-HBs, human polyclonal anti-HBs and anti-tetanus toxoid antibody on the HBsAg-induced proliferation of the HBC-4 T cell clone.

Figure 5 illustrates that monoclonal anti-HBs enhance the proliferative response of the HBC-6 cell line to different preparations of HBsAg.

Figure 6 illustrates the effect of monoclonal antiHBs on the proliferation of the HBC-6 cell line induced by two different HBsAg preparations.

-8-

Figure 7 illustrates that antigen binding fragments of anti-HBs do not enhance the proliferative response of HBsAg-specific T cell clones.

Best Mode of Carrying Out the Invention

Like most soluble and particulate antigens, synthesis of antibodies to HBsAg is probably regulated by T lymphocytes. In order to study the response of human T lymphocytes to antigen at the cellular level, several human T lymphocyte clones which proliferate to HBsAg in an antigen-specific manner were developed. The clones were isolated from cultured peripheral blood mononuclear cells (PBM) of hepatitis B vaccine recipients by in vitro antigen stimulation in the presence of the lymphokine interleukin-2. Details of the procedure for isolating the T cell clones are described infra. Briefly, mononuclear cells were obtained from the peripheral blood of hepatitis B vaccinees and were examined for various immune responses to HBsAg in vitro. PBM from most vaccinees did not proliferate to a great extent when exposed to HBsAg. However, lymphocyte proliferation to HBsAg could be increased substantially by maintaining the cells in the culture with a T cell growth supplement (a partially processed supernatant of activated T lymphocytes containing the lymphokine interleukin- 2). In this manner, cell lines were established. From an HBsAg-reactive lymphocyte line derived from the donor whose cells exhibited the greatest proliferative response to HBsAg, T cells clones were established by standard techniques. Most of the resulting T cell clones were HBsAg-specific; they proliferated when exposed to HBsAg (ad or ay subtype) but not to unrelated antigens (tetanus toxoid or influenza A virus). All clones exhibited a helper/inducer surface phenotype (OKT4$^+$, OKT8$^-$) indicating that they might belong to the helper T cell subtype. At least two of the clones actually provided help in the production of anti-HBsAg antibodies by autologous B cells.

The effect of polyclonal human anti-HBs on the proliferative response of two of the HBsAg-specific human T lymphocyte clones (designated HBC-6 and HBC-13) was examined. The two clones had been maintained in culture with HBsAg and interleukin-2 for about 3 months. Both of these clones exhibited helper function, that is, they promoted anti-HBs production by B cells. Also, they were shown to secrete interferon-gamma and B cell growth factors when stimulated by antigen. Polyclonal human anti-HBs was purified from plasma of hepatitis B vaccine recipients by affinity chromatography with an immunoadsorbent containing HBsAg.

Polyclonal anti-HBs dramatically increased the proliferative response of the human T lymphocyte clones HBC-6 and HBC-13 to HBsAg. At a constant concentration of HBsAg, the increase in proliferation caused by anti-HBs was dose related. The greatest increase in proliferation was attained at antibody concentrations in excess of antigen concentration. Further, in the presence of a constant amount of anti-HBs, a maximum proliferative response to HBsAg can be elicited at antigen concentrations 10-100 times lower than concentrations required in the absence of the anti-HBs.

The potentiation of proliferation by polyclonal anti-HBs is antigen-specific. Antibody not specific for HBsAg produced no enhancement of proliferation. For example, purified human anti-tetanus toxoid antibodies caused no potentiation of HBsAg-induced proliferation of T cell clones, but instead caused a slight inhibition of the proliferative response.

Murine monoclonal antibodies against HBsAg were examined for the ability to enhance proliferation of the human T cell clones. Murine anti-HBs producing hybridomas were prepared by standard somatic cell hybridization techniques. Wands, J.R. and Zurawski, V.R. (1981) Gastroenterol. 80, 225-232. Monoclonal antibodies were obtained by injecting the hybridomas into the peritoneal cavity of mice and isolating the antibody-rich ascites fluid.

The HBsAg-specific murine monoclonal antibodies increased antigen-induced proliferation of the T cell clones. A series of twelve different monoclonal antibodies was tested; all antibodies induced a significant enhancement of antigen-induced proliferation. However, a differential effect was observed for antibodies of different isotypes. In general, monoclonal anti-HBs of the IgG class exhibit a greater potentiating effect than did monoclonal anti-HBs of the IgM class with only one exception. A monoclonal antibody of the $IgG_{2b}$ class (the only antibody of this type tested) was not as effective as $IgG_1$ and $IgG_{2a}$ monoclonal antibody and was about as effective as the IgM monoclonal antibody. Notably, murine monoclonal $IgG_1$ and $IgG_{2a}$ antibody were superior to polyclonal human anti-HBs.

The enhancement of antigen-induced response by monoclonal antibody was found to occur with preparations of both soluble and particulate forms of HBsAg. HBsAg prepared from plasma of hepatitis B vaccines consists of the hepatitis B viral envelope, a particulate antigen. The results discussed thus far were obtained with this HBsAg preparation. An enhanced response was also observed with two preparations of HBsAg which contained the soluble HBsAg molecule.

Although antigen-induced proliferation is a good measure of T cell activation, it does not measure T cell effector or regulatory function. In order to determine the effect of antibody on antigen inducement of effector function of T lymphocytes, monoclonal anti-HBs was tested for its effects on antigen-induced production of interferon-gamma by the HBsAg-specific T cells. Murine monoclonal anti-HBs significantly enhanced interferon-gamma secretion of three HBsAg specific T cell clones, indicating that antibody enhances antigen-induced effector function, as well as proliferation of antigen-specific T lymphocytes.

In addition to enhancing of the response of clonal T cells, HBsAg-anti-HBs complexes also increased the proliferative response of freshly obtained human lymphocytes. This enhancement probably entails the collective response of various T cell clones having surface receptors specific for different epitopes of HBsAG.

The potentiation of antigen induced proliferation by antigen-antibody complexes is not unique to the HBsAG system. Both polyclonal and mouse monoclonal anti-tetanus toxoid antibodies enhanced antigen-induced proliferative response of human T cells specific for tetanus toxoid.

The experiments described herein demonstrate that antibodies function not only as effector molecules in binding and neutralizing antigen but also as molecules which influence antigen-activation of T cells. The mechanism by which antibody enhance the antigen-induced response of the T lymphocytes has not been elucidated. A possible explanation is related to the ability of antibody to polymerize and aggregate the antigen with which they react. In polymer form, the number of antigenic determinants or epitopes per polymer molecule (valence) is higher than in the monomer form. It has been established that antigenic valence is directly proportional to the apparent binding affinity of the surface receptors for the antigen. See. E. Celis et al. (1977) Immunochem. 14, 553. Thus, the polyvalent immune complexes may bind more strongly to cell surface antigen receptors than the antigen itself, and, as a result of this, may evoke a more vigorous T cell response.

Antigen and antigen-antibody complexes probably do not bind directly to the antigen receptor on the T lymphocyte surface because interaction with the T cell receptor is mediated by the antigen presenting cells such as macrophages and monocytes. Another possible explanation for the enhanced T cell response induced by antigen-antibody complexes is that antigens aggregated by antibody may be more easily captured by these cells than soluble, unaggregated antigens. Macrophages and monocytes have surface receptors specific for the Fc portion of IgG antibody (the portion of the antibody which does not bind antigen).

The Fc portion of IgG anti-HBs is required for antibody potentiation of proliferation. In experiments with antibody fragments, the $F(ab')_2$ and Fab fragments of murine monoclonal anti-HBs were incapable of evoking enhancement of HBsAg-induced proliferation of the T cell clones. Because these antibody binding fragments should aggregate antigens like intact antibodies, and further because IgM monoclonal antibody should do the same, aggregation of antibody per se may not be the major factor accounting for potentiation of T cell proliferation. It seems that Fc receptors on antigen presenting cells may be involved.

Another phenomenon which may contribute to the enhancement of T cell response by antigen-antibody is binding of the complexes to complement receptors on monocytes. Complexes may bind to monocytes through complement receptors as well as Fc receptors.

-14-

The results discussed herein demonstrate that antigen-antibody complexes potentiate antigen-induced proliferative response and effector function of human T lymphocytes in vitro. Accordingly, antigen-antibody complexes can be used to achieve more efficient in vitro immunization of T lymphocytes. Procedures of immunizing lymphocytes in culture are essential steps in a number of immunological techniques. An example is formation of antigen-specific T cell and B cell clones or lines. Clonal populations of antigen-specific T lymphocytes can be used to investigate T cell response to antigen at the cellular level. Further, these cells can be a source of various T cell produced factors such as lymphokines which have important research and therapeutic uses. Clonal populations of B cells can be used for the production of antibody.

In the production of antigen-specific T cell clones, antigen-antibody complexes can be used to activate and expand the antigen-specific T cells within a population of lymphocytes. Lymphocytes may be obtained as peripheral blood mononuclear cells from a donor. In other preparations antigen presenting cells may have to be added. After activating the lymphocytes, they may be fused with a neoplastic lymphoid cell such as a T lymphoma by standard somatic cell hybridization techniques. Then the hybrid cells can be cloned and the desired antigen-specific hybrid clone selected.

Antigen-antibody complexes can also be used to produce antibody-secreting B lymphocyte clones. For example, antigen-antibody complexes can be used to augment T helper cell population within a lymphocyte culture. The activated helper T cells, in turn, expand the population of B cells that secrete antigen-specific antibody. Thereafter, the B cells may be fused with a neoplastic lymphoid cells to produce hybrid cells. The hybrid cells are then cloned and screened for production of desired antibody. Instead of hybridizing the activated B cells, the activated B cells may be transformed by a virus such as the Epstein-Barr virus or by other suitable procedures.

Antigen-antibody complexes are especially useful for enhancing the T cell response to antigens which are only marginally immunogenic. Small molecules, such as peptides, often elicit only a weak immunogenic response. By complexing these molecules to antibody specific for them, immunogenicity can be enhanced. In some instances, however, it may be difficult or impossible to obtain antibody specific for a weakly or non-antigenic substance. A strategy for obtaining an immune response to such substances is to couple the substance to a carrier molecule for which antibody is readily obtainable. Examples of suitable carriers are proteinous molecules such as albumin or immunoglobulin and carbohydrate polymers such as Ficoll. The complexes of antigen coupled to the carrier and antibody specific for the carrier can be used to induce an immune response against the antigen.

Antigen-antibody complexes for in vitro immunization can be formed with polyclonal or monoclonal antibody. Monoclonal antibody of the IgG class is preferred because antibody of this class evokes the most potent enhancement of antigen-induced T cell response. Importantly, the monoclonal antibody may be heterologous, that is, it need not be obtained from the same species as the cells which are to be immunized. Mouse, goat or rabbit antibody can be used to immunize cells of different species. For example, as described supra, complexes can be formed with antigen and murine monoclonal antibody specific for the antigen to immunize human T cells. This is particularly advantageous because procedures for forming murine monoclonal antibodies are well established.

To form the antigen-antibody complexes, antigen and antibody specific therefor are admixed in solution, for example phosphate buffered saline. The immune complexes form spontaneously. Preferably, complexes are formed with antibody in excess of antigen. Although some enhancement of T cell response is observed with antigen-antibody complexes formed at molar equivalence, the most potent response is evoked with complexes formed with greater concentration of antibody. The preferred molar ratio of antigen to antibody ranges from about equivalence to about 1/100. After the complex is formed, it is added to the cell culture medium.

Antigen-antibody complexes can be formed with particulate antigens such as bacteria, viruses and macrocomponents of cells, and soluble antigens such as proteins, peptides, glycoproteins and carbohydrates. Antigens of particular interest are tumor-associated antigens, synthetic antigens and antigens derived from recombinant microorganisms or mammalian cells.

As demonstrated by the studies conducted in vitro, antibody exerts a profound potentiating effect on the response of antigen-specific T lymphocyte to antigen. Antibody might also play an important role in the natural immune response to antigen by amplifying T cell response in vivo. Most likely, this amplifying effect of antibody occurs in secondary immune responses when some amount of specific antibody is present in the blood. In fact, secondary immunization is known to evoke a greater immune response than primary immunization. The presence of antibody might account in part for this natural enhanced response.

A significant aspect of this invention is that immune complexes formed with monoclonal antibody may be used to augment T cell clonal expansion in vivo. This provides for improved production of vaccines to infectious disease and of immunogen compositions useful for therapy of chronic disease and cancer. Antigen-monoclonal antibody complexes can be used to increase the efficiency of vaccine compositions or to enhance the immunogenicity of weakly antigenic substances.

-18-

Monoclonal antibody provides a number of important advantages over polyclonal antibody. For one, complexes formed with monoclonal antibody of the $IgG_1$ and $IgG_{2a}$ subclasses elicit the greatest enhancement of T cell response. Also, monoclonal antibody of preselected specificity can be produced in unlimited supply by well-known techniques. Finally, the monoclonal antibody need not be homologous to the species immunized. When obtainable, homologous monoclonal antibody is desirable because interspecies response against immunoglobulin is eliminated. However, hybridoma technology has not been perfected in many species, most notably humans; and therefore it is not always possible to obtain homologous antibody from these species.

Immunogen compositions can be formed with antigen and monoclonal antibody specific for the antigen. The immune complex should be formed with an excess of the monoclonal antibody, ranging from about equivalence to about 100 times the concentration of antigen. Monoclonal $IgG_1$ or $IgG_{2a}$ antibody is employed because monoclonal antibody of these classes elicit the most pronounced T cell proliferative response to antigen. As with conventional immunogen composition, the complexes may be administered subcutaneously or intramuscularly in a physiologically acceptable vehicle.

This method of immunological stimulation in vivo has a number of important advantages. Effective vaccination can be achieved by immunizing with antigen in much smaller amounts when complexed to

-19-

antibody than when uncomplexed. Thus the amount of antigen required for immunization can be reduced. This may allow more widespread use of vaccines containing antigen which are difficult and costly to prepare. The method can be especially useful when the antigen is toxic at the concentration normally required for effective immunization. By reducing the amount of antigen needed for vaccination, the risk of toxicity is reduced.

As in the case of in vitro immunization, protocols may be devised to enhance the immune response of marginally or non-antigenic substances for which specific antibody is difficult to obtain. For example, a small peptide antigen may be coupled to a protein carrier and immune complexes may be formed with antibody to the carrier. In this manner, vaccines employing synthetic peptides and products of recombinant DNA technology may be made more efficient.

The invention is further illustrated by the following examples.

## EXAMPLES

Example 1. Isolation of HBsAg-specific T lymphocyte clones.

Antigens

Plasma containing HBsAg was collected from chronic hepatitis B carriers. HBsAg was purified by affinity chromatography using Sepharose 4B conjugated with mouse monoclonal antibody 5D3. This antibody reacts with both ad and ay serotypes of HBsAg.

-20-

HBsAg ad and ay serotypes were purified by incubating the separate plasma with the immunoadsorbent overnight and by eluting the HBsAg with 0.2 M sodium acetate, pH 4.8, after washing the columns thoroughly with phosphate-buffered saline (PBS, 0.15 M NaCl, 0.015 M $PO_4$, pH 7.4). The eluted material was dialyzed extensively against PBS, then concentrated in the dialysis tube by absorbing water with polyethyleneglycol powder (Calbiochem, San Diego, CA) and finally filter-sterilized with a 0.22 um pore membrane. HBsAg derived from the tissue culture supernatant of the human hepatoma line PLC/PRF/5 (obtained from Dr. Daniel Shouval, Hadassah University, Jerusalem) was purified by the same affinity chromatography procedure. Protein concentration was determined by the BioRAD protein assay with bovine serum albumin used as a standard. To avoid aggregation, the HBsAg was stored at 4°C until used.

A different HBsAg preparation (kindly provided by Dr. William J. McAleer, Merck Sharp and Dohme Research Laboratories, West Point, PA) was also tested in the present studies. The Merck HBsAg was prepared from 22-nm HBsAg particles, which were purified from plasma pools by ammonium sulfate concentration, isopyknic ultracentrifugation in sodium bromide, and rate-zonal ultracentrifugation in sucrose. These noninfectious 22-nm particles were then treated with 1 mg/liter pepsin (pH 2.1) at 37°C for 18 hours followed by 8 M urea for 4 hours. The purified HBsAg was dialyzed extensively to remove the urea and was filter-sterilized. The procedure was the same as that employed for manufacturing the commercial Heptavax B vaccine except
that the vaccine was finally treated with a 1:4000
formaldehyde solution at 37°C for 72 hours.

Radioimmunoassays were performed in all HBsAg
preparations to test for the presence of mouse and
human immunoglobulins. None of the HBsAg purified
by affinity chromatography with 5D3 immunoadsorbent
contained any detectable amounts of mouse immunoglobulins (less than 0.1 ng per 10 ug of protein),
indicating that the 5D3 monoclonal antibody was not
removed from solid support by the acid elution. The
HBsAg obtained from human plasma contained large
amounts (20 to 50%) of human immunoglobulins. Both
the PLC/PRF/5-HBsAg and the Merck-HBsAg preparations
did not contain any detectable amounts of human
immunoglobulins (less than 0.1 ng per 10 ug of
protein).

Tetanus toxoid was purchased from the State
Laboratory, Commonwealth of Massachusetts, Boston,
MA. Influenza A virus of Bangkok strain (formalin-
inactivated) was obtained from Connaught laboratories, Inc., Swiftwater, PA. The commercially
obtained antigens were dialyzed against PBS to
remove the thimerosal before use in tissue culture.

Isolation of HBsAg-reactive T lymphocytes.

One hundred milliliters peripheral blood from
each of 10 donors who had received three doses of
hepatitis B vaccine (Merck Institute of Therapeutical Research, West Point, PA) were obtained 7 days
after the last immunization. All individuals studied did not have any anti-HBs in their sera before the first immunization, as measured by a commercial radioimmunoassay (AUSAB, Abbott Laboratories, Chicago, IL). PBM were isolated by centrifuging the heparinized blood on a Ficoll/diazotrizoate density (d = 1.007) gradient (Pharmacia, Piscataway, NJ). For in vitro stimulation, PBM were incubated in a T-75 tissue culture flask at $5 \times 10^5$/ml with 20 ug/ml of HBsAg in 25 ml RPMI 1640 (GIBCO, Grand Island, NY) containing 2 mM -glutamine, 25 mM HEPES, 50 ug/ml gentamycin and $5 \times 10^{-5}$ M 2-mercaptoethanol (complete medium) that was supplemented with 10% human AB serum (Flow Laboratories, Inc., McLean, VA). After incubation at 37°C in an 8% $CO_2$/air humid environment for 6 days, the cells were centrifuged on a Ficoll/diazotrizoate gradient. The live cells were then resuspended at $1 \times 10^5$/ml in complete medium with 10% fetal celf serum (FCS, HyClone Sterile systems Inc., Logan, UT) instead of the human serum. Also included in the culture medium was lectin-free T cell growth supplement (TCGS) from Meloy Laboratories, Springfield, VA at 10% final concentration. TCGS is concentrated culture supernatant of activated human PBM and presumably contains interleukin 2 (IL 2) and other factors required for continual propagation of T cells. After incubation in 24-well plates (Costar, Cambridge, MA) for 1 week, the cells were resuspended at $1 \times 10^5$/ml and restimulated with 20 ug/ml of HBsAg in the presence of $5 \times 10^5$ autologous irradiated (2000 rad) PBM and 5% TCGS for 3 days. Then 1

-23-

vol of complete medium with 10% TCGS was added per
volume of cell culture and the cells were incubated
for four additional days.  At this point an antigen-
induced proliferative assay was performed, and the
lymphocytes from one of the donors (DV) were selected for further study and for cloning.

Cell cloning

HBsAg-reactive lymphocytes were cloned by
limiting dilution in 96 flat-bottomed well plates
(Costar) at one cell per well in the presence of 20
ug/ml of antigen, 10% TCGS, 20% FCS, and $5 \times 10^5$
autologous irradiated PBM per milliliter.  After 7
days of incubation all the wells were fed with
complete medium containing 10% TCGS.  By day 12,
clones of growing cells were easily identifiable
with a low power inverted microscope.  In a typical
cloning experiment, about 15% of the wells contained
clones.  The cells from each one of these wells were
transferred into three wells of 96-well plates,
adding fresh medium with 10% TCGS.  After 5 days,
the cells in the three wells were transferred into a
single well of a 24-well plate with HBsAg and
irradiated-autologous PBM, and at the same time were
tested for antigen reactivity and studied further.

Proliferation assays

When fresh PBM were tested, $2 \times 10^5$ cells were
incubated in medium with 10% human AB serum for 5 to
6 days with different antigen concentrations in each
well of 96-well (flat-bottomed) plates.  When
antigen-stimulated lymphocyte lines or clones were
tested, they were incubated at $1 \times 10^4$ cells/well

for 3 days in the presence of 5 x $10^5$/ml autologous, irradiated PBM and with medium containing 10% FCS and antigens. All assay cultures were pulsed for the last 15 to 20 hours with 1 uCi of [$^3$H] thymidine (New England Nuclear, Boston, MA) per well. After the cells were harvested and washed on glass-fiber filters with a cell harvester (Bellco, Vineland, NJ), the incorporation of radiolabel into DNA was determined by scintillation spectroscopy using a Beckman beta counter. Results are presented either as the mean counts per minute (cpm) of two or three replicate determinations, the standard error of the mean, or as the "proliferative index". Proliferative index is the ratio of the mean cpm incorporated in the presence of antigen to the mean cpm incorporated in the absence of antigen. Because the proliferative index can be greatly affected by high cpm in the background, the maximal cpm incorporated are also shown in parentheses to reveal the proliferative activity of the cells.

Determination of cell surface antigens

2 x $10^5$ washed and sedimented cells were incubated with 100 ul of the appropriate dilution (usually 1/100) of the murine monoclonal antibodies OKT3, OKT4 and OKT8 (ortho Diagnostic Systems, Raritan, NJ) for 30 minutes on ice. Cells were washed twice with RPMI 1640 containing 5% FCS by centrifugation at 800 x G for 5 minutes in the cold, resuspended in 50 ul of a 1/10 dilution of fluorescein isothiocyanate-labeled goat anti-mouse IgG (Meloy) and incubated again for 30 minutes in ice.

-25-

After washing three times in the cold, the cells were subjected to fluorescence-flow cytometric analysis in a FACS IV cell sorter (Becton Dickinson, Mountain View, CA). A mouse monoclonal antibody against tetanus toxoid was included in all experiments as a negative control.

Enrichment of HBsAg-specific cells

Cells that bound to HBsAg were selected from PBM from hepatitis B vaccine recipients by panning the cells onto HBsAg-coated plastic petri dishes, a procedure similar to that described by Mage et al. and Wysocki and Sato. (references) Bacteriologic plastic petri dishes (Fisher) of 10-cm diameter were coated overnight at 4°C with 15 ml of HBsAg at 10 ug/ml in PBS. The petri plates were washed four or five times with PBS, and $5 \times 10^7$ PBM in 3 ml of RPMI 1640 with 5% FCS, 25 mM HEPES, and 1 mM EDTA were added to each plate. After the plates were placed at 4°C for 30 minutes, they were gently swirled and allowed to sit for an additional 30 minutes. The non-attached cells were removed by gently washing the plates three times, and the antigen-enriched cell population was harvested by vigorously flushing the plates with complete medium by using a 5-ml pipet. Both cell populations that were enriched for and depleted of antigen-binding cells were washed with complete medium before use.

In vitro antibody production assay

Different numbers of T lymphocytes were incubated at 37°C with $5 \times 10^4$ HBsAg-enriched cells (containing HBsAg-binding cells) and $5 \times 10^4$ cells

that had been depleted of antigen-binding cells (presumably containing antigen-presenting cells) in 200 ul of complete medium with 4 ng/ml of HBsAg in 96-well (round-bottomed) plates (Costar). After a 6-day incubation, the antigen was removed by washing the plates two times with fresh complete medium and the cells were resuspended in their original volume and incubated for six additional days. The amounts of anti-HBs produced were determined with a commercial radioimmunoassay kit. (AUSAB, Abbott Laboratories).

Results

PBM obtained from 10 individuals who had received three doses of the hepatitis B vaccine were assayed for their ability to proliferate in response to HBsAg in vitro. In only one case was vigorous proliferation observed; however, if the cells were stimulated in vitro with HBsAg and grown in the presence of TCGS, significantly greater proliferation of cells from some donors was observed. Of the ten donors, cells from three showed substantial proliferation after in vitro antigenic stimulation (DV, BS, and CB) whereas cells from only one donor (SM) demonstrated a good response without stimulation. The lymphocytes from the remaining six donors made only marginal proliferative response to high concentrations (75 to 150 ug/ml) of HBsAg when assay were performed with fresh PBM or with in vitro antigen-stimulated lymphocytes (data not shown).

Antigen-reactive lymphocytes from donor DV, which displayed the highest proliferative index in response to HBsAg, were cloned by limiting dilution. The clones were assayed for proliferative response to HBsAg and certain unrelated antigens. The results presented in Table 1 were obtained with 20 lymphocyte clones (HBC) and the antigen-reactive cell line (HBL). All of the clones proliferated in an antigen-specific manner. Significantly higher [$^3$H]thymidine incorporation was obtained when the cells were incubated in the presence of HBsAg (either ad or ay serotype) or TCGS, but not other unrelated antigens.

– 28 –

Table I

<u>Proliferative Responses of HBsAg-Induced</u>
<u>T Cell Clones to Various Antigens</u>

<u>Clone</u>                    <u>Cell Proliferation (cpm)</u>

| | HBsAg(ad) | HBsAg(ay) | Tetanus toxoid | Influenza A | TCGS | Medium |
|---|---|---|---|---|---|---|
| HBC-1 | 26676 | 21297 | 120 | 710 | 13209 | 272 |
| HBC-3 | 20446 | 14561 | 1705 | 1543 | 11470 | 1035 |
| HBC-4 | 22916 | 15958 | 255 | 318 | 24750 | 301 |
| HBC-5 | 12558 | 11035 | 981 | 1151 | 5251 | 138 |
| HBC-6 | 21667 | 15801 | 331 | 619 | 11177 | 552 |
| HBC-7 | 13618 | 8623 | 632 | 714 | 11868 | 667 |
| HBC-10 | 12655 | 10730 | 203 | 331 | 12512 | 642 |
| HBC-12 | 20265 | 16605 | 1065 | 5790 | 10677 | 4959 |
| HBC-13 | 33538 | 32440 | 2304 | 5700 | 14196 | 4564 |
| HBC-15 | 12424 | 11602 | 2310 | 1984 | 11134 | 769 |
| HBC-16 | 14447 | 13914 | 835 | 1198 | 8512 | 907 |
| HBL | 26448 | 25375 | 1114 | 3081 | 14669 | 2263 |

The antigen specificity of some of the HBsAg-reactive T lymphocyte clones was examined by testing the ability of different HBsAg preparations to induce proliferation.    The HBsAg preparations tested were HBsAg purified from culture supernatants of the PLC/PRF/5 human hepatoma line (PLC/PRF-HBsAg) and HBsAg obtained from Merck, Sharpe and Dome Laboratories (MSD-HBsAg). All of the HBsAg preparations triggered proliferation response of the clones with similar efficiency. The clones did not show any significant proliferative response to human serum proteins.

The proliferative response of clones 1-16 to varying concentrations of HBsAg was examined. Although all clones were antigen-reactive, a wide range of proliferation indices, ranging from 9 to 110, was observed at the highest HBsAg antigen concentration (75 ug/ml). At this concentration of antigen, [$^3$H]thymidine uptake always exceeded 12,000 cpm.

Clones 1-16 were analyzed by cytofluorography for the common T cell surface markers with several monoclonal antibodies. All of the clones were OKT3$^+$, OKT4$^+$, OKT8$^-$, indicating that they may belong to the helper/inducer class (data not shown). The capacity of these cells to stimulate anti-HBs production was assessed by an _in vitro_ helper assay. As shown in Table II, clones HBC-6 and HBC-13 stimulated anti-HBs production by a cell population enriched for HBsAg-binding B lymphocytes in 12-day cultures.

- 30 -

## Table II

### Helper Activity of HBsAg-specific T Cell Clones For the Antibody Response Against HBsAg[a]

| Number of T Cells per Culture | Anti-HBs Production[b] (cpm) | | | | | | | | | |
|---|---|---|---|---|---|---|---|---|---|---|
| | HBC-1 | HBC-3 | HBC-4 | HBC-5 | HBC-6 | HBC-7 | HBC-10 | HBC-12 | HBC-13 | HBC-15 |
| $10^4$ | 145 | 141 | 123 | 186 | 122 | 159 | 148 | 164 | 10,875 | 214 |
| $10^5$ | 126 | 121 | 150 | 156 | 613 | 118 | 117 | 152 | 495 | 335 |
| $10^2$ | 155 | 141 | 194 | 176 | 162 | 136 | 148 | 151 | 214 | 145 |

[a]Different numbers of HBsAg-reactive clone cells were incubated with $1 \times 10^5$ cells enriched for antigen-binding cells for 12 days in a final volume of 200 ul of media. The antigen-enriched cells alone (without any clone cells) gave a result of $149 \pm 10$ cpm.

[b]Anti-HBs was determined with a radioimmunoassay kit (AUSAB, Abbott). The negative control gave 166 cpm. According to the statistical data analysis method described in AUSAB assay procedure, experimental values higher than 2.1 times the value of negative control are considered to indicate the presence of anti-HBs. Thus, in the present experiment, results higher than 350 cpm (underlined) were considered positive. Each result represents the mean of duplicate derminations.

The helper activity of clone HBC-13 cells was examined further with large numbers of replicate cultures and different concentrations of HBC-13 cells. Although the amount of anti-HBs varied greatly, statistically significant amounts of anti-HBs were produced by antigen-enriched B cells when HBC-13 cells were present in the cultures. HBC-13 cells provided the best help at a cell concentration of 10% (percentage of total cells in culture.)

Example 2. Effect of anti-HBs on antigen-induced proliferation of HBsAg-specific T cell clones.

Antibodies

Anti-HBs were purified from plasma of hepatitis B vaccinees by affinity chromatography with crosslinked agarose (Affi-Gel 10, from Bio-Rad, Richmond, CA) coupled with HBsAg. The eluted anti-HBs were negative for the presence of HBsAg by a sensitive radioimmunoassay (less than 0.1 ng of HBsAg per 200 ug of anti-HBs).

Cell proliferation assays

$2 \times 10^4$ HBsAg-specific T cells were mixed with $10^5$ autologous irradiated PBM in the presence of different concentrations of HBsAg and antibodies in a final volume of 200 ul of tissue culture medium in each well of 96 flat-bottom well trays. The proliferation assays were incubated at 37° for 3 days and each culture well was pulsed for the last 15-20 hours with 1 uCi of [³H]-thymidine (New England Nuclear, Boston, MA). The cultures were harvested and washed using a Skatron cell harvester
(Sterling, VA) and the amount of radiolabel incorporated into DNA was determined by liquid scintillation spectroscopy.  Results of "cell proliferation"
are expressed as the mean counts per minute (cpm) of
duplicate determinations.  The standard error of the
mean was always below 10% of the value of the mean.

Antigens

HBsAg was purified from plasma of chronic
carriers of hepatitis B virus by affinity chromatography in Affi-Gel 10 coupled with the mouse monoclonal antibody 5D3, which specifically reacts with
HBsAg.  The purified HBsAg preparations were dialysed extensively against phosphate buffered saline
(PBS; 0.1 M NaCl, 0.01 M, $PO_4^-$, pH 7.4) and filter-
sterilized with a 0.2 um membrane.

HBsAg-specific T cell lines and clones

Isolation and characterization of the HBsAg-
specific T cell clones are described in Example 1.

Cell Proliferation Assays

Cell proliferation was measured by incubating
$2 \times 10^4$ cloned T cells and $10^5$ irradiated autologous
PBM with different concentrations of HBsAg and
anti-HBs in 96-flat-bottom well trays (Costar) in a
final volume of 200 ul of RPMI-1640 tissue culture
medium that contained 10% fetal calf serum, 25 mM
HEPES, 5 mM L-glutamine $5 \times 10^{-5}$ M 2-mercaptoethanol.
After a 54 hour incubation at 37°C in a 5% $CO_2$-95%
humid air environment, each culture was pulsed with
1 uCi of [$^3$H]-thymidine for 18 hours under the same
conditions.  The amount of radiolabel incorporated into DNA was determined by liquid scintillation spectrography after harvesting the cultures onto glass-fiber filters.

Results

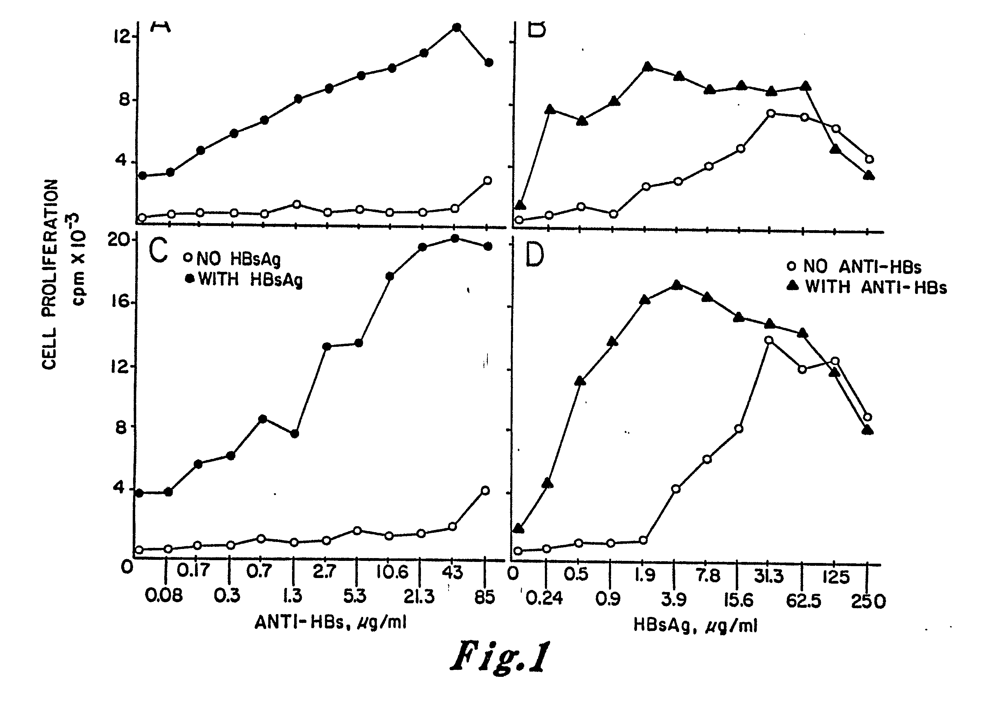

The effects of different amounts of anti-HBs on antigen-mediated proliferation of HBsAg-specific T cell clones are illustrated in Figure 1. Clones HBC-6 (Panel A) and HBC-13 (Panel C) were incubated with various amounts of anti-HBs in the presence of 2 ug/ml of HBsAg (●), and in the absence of HBsAg (○). The proliferation of HBC-6 (Panel B) and HBC-13 (Panel D) induced by different concentrations of HBsAg was determined in the presence of 20 ug/ml of anti-HBs (▲) and in the absence of antibodies (○). Each point represents the mean of duplicate determinations and the standard error of the mean for each point was below 10% of the mean.

Figure 1A and 1C show that anti-HBs dramatically increase the proliferative response of T cell clones, HBC-6 and HBC-13, to HBsAg in a dose related manner. Anti-HBs alone produces a small but significant increase of the proliferative response of both clones. As shown in Figure 1B and 1D in the presence of a constant amount of anti-HBs (20 ug/ml), the maximum response to HBsAg could be achieved at antigen concentrations 10-100 times lower than antigen concentrations in the absence of anti-HBs.

-34-

Example 3.  Effect of antibodies of different antigenic specificities on HBsAg-induced proliferation of antigen-specific T cell clones.

Purification of anti-HBs and of HBsAg and determination the degree of cell proliferation were conducted as described in Example 1.  Anti-tetanus toxoid antibodies were purified from plasma obtained from volunteers recently immunized with tetanus vaccine by affinity chromatography with Sepharose 4B (Pharmacia, Piscataway, NJ) coupled with tetanus toxoid (State Laboratory, Commonwealth of Massachusetts, Boston, MA).

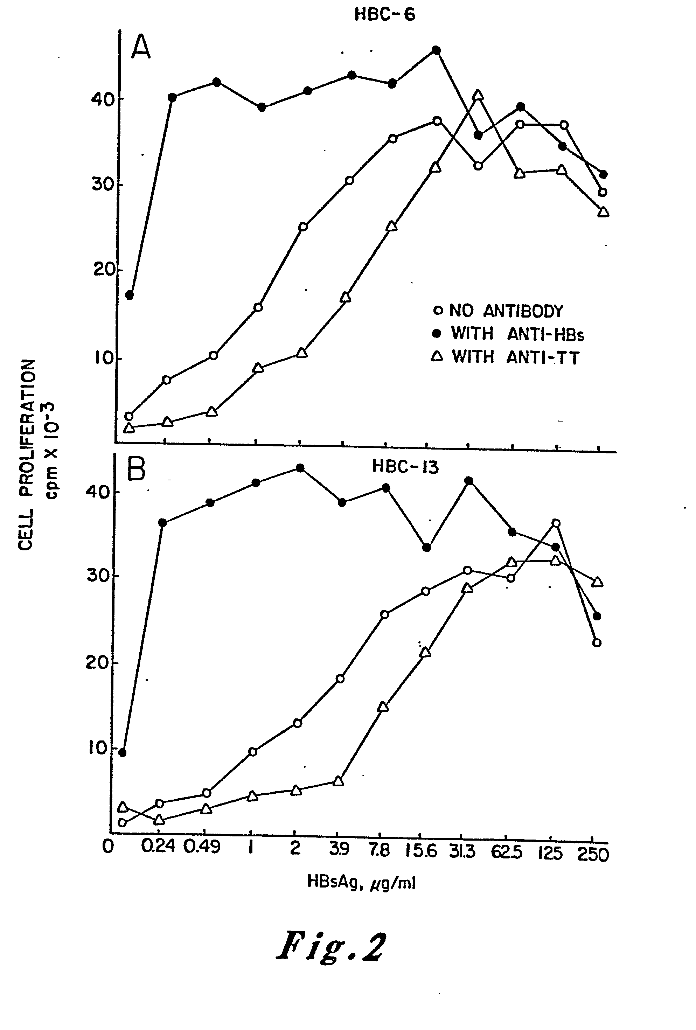

Figure 2 shows the effect of anti-HBs and anti-tetanus toxoid antibody on antigen-mediated proliferation of the HBsAg-specific T cell clones. HBC-6 (Panel A) and HBC-13 (Panel B) cells were incubated with different concentrations of HBsAg in the presence of 20 ug/ml of anti-HBs (●) or anti-tetanus toxoid antibody  (△), or in the absence of antibody (O).  Each point represents the mean of duplicate determinations and standard error of the mean for each point was below 10% of the mean.

The anti-tetanus toxoid antibody inhibited rather than enhanced the proliferative response. This indicates that the effect of anti-HBs on the T cell response to HBsAg is antigen specific.

Example 4.  Effects of murine monoclonal antibodies in the HBsAg-induced proliferation of clone HBC-6. Antibodies

Polyclonal human anti-HBs were obtained as described in Example 1. Mouse monoclonal anti-HBs were obtained from hybridoma cells produced by somatic cell hybridization techniques previously described. Wands, J.R. and Zurawski, V.R., Jr. (1981) Gastroenterol. 80, 225-232. Ascites fluid containing 5-20 mg of specific antibody per ml were used in most experiments. The mouse monoclonal antibody B2TT specific for tetanus toxoid was also used [Zurawski, V.R., Jr. et al. (1980) Fed. Proc. 39, 4922 (abs.)]. The mouse monoclonal anti-HBs A5C3 was purified from ascites fluid by affinity chromatography on protein A Sepharose (Sigma Chem. Co., St. Louis, MO).

$F(ab')_2$ and Fab fragments of A5C3 were prepared by protease digestion with pepsin and papain respectively as described [Chang, T. et al. (1982) J. Immunol. 128, 585-589]. The $F(ab')_2$ and Fab fragments of A5C3 were further purified by removing the undigested IgG molecules and Fc fragments with protein-A Sepharose. High pressure liquid chromatography analysis of the whole antibody and fragments revealed a single peak of the appropriate molecular weight for each preparation. Protein concentration was determined as in Example 1. Ascites fluids, purified anti-HBs and fragments of anti-HBs were all filter-sterilized with a 0.2 um membrane before use in tissue culture.

HBsAg-specific T cell clones

Details for isolation of the HBsAg-reactive T cell clones are given in Example 1.

## Cell proliferation assays

Cell proliferation assays were performed as described in Example 2.

## Production and determination of IFN-gamma

HBsAg-reactive T cells were incubated at $1 \times 10^5$ cells/ml with $5 \times 10^5$/ml autologous-irradiated PBM in the presence and absence of anti-HBs and HBsAg for 48 hours in 24 flat-bottom well trays (Costar). Supernatants were removed and filtered through a 0.2 um membrane and assayed for IFN-gamma using a sandwich immunoradiometric assay. Quantitation of IFN-gamma was performed using a standard curve with DEAE-purified gamma-IFN from mitogen activated human PBM. The sensitivity of this assay is approximately 0.1 NIH U/ml.

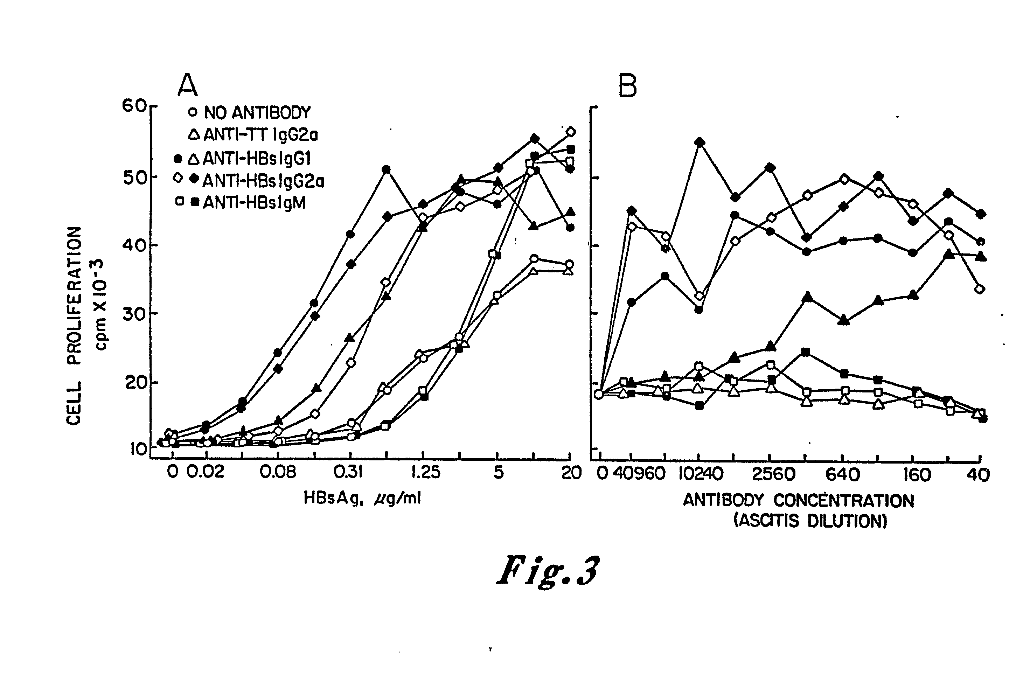

Twelve mouse monoclonal antibodies were examined for their effect on antigen-induced proliferation and immune factor production of three HBsAg-specific T cell clones. One of the clones, HBC-6, was shown to promote production of anti-HBs by B cells. The other two, HBC-1 and HBC-4, were OKT4[+] and OKT8[-] but their function is unknown. Figure 3 shows results obtained with six of the monoclonal antibodies. In panel A, cell proliferation was determined with different concentrations of HBsAg and a constant concentration of each antibody. In panel B different concentrations of the same antibodies used in panel A were tested for their effects on proliferation of HBC-6 at constant concentration of HBsAg (1 ug/ml). The monoclonal anti-HBs used

were: ● A2C6 (IgG$_1$); ▲ H2C4 (IgG$_1$); ◇ A5C3 (IgG$_{2a}$); ◆
A5C11 (IgG$_{2a}$); ☐ H2F11 (IgM); ■ H5D3 (IgM). Monoclonal anti-tetanus toxoid (TT) antibody B2TT (IgG$_{2a}$)
was included as a negative control (△) and proli-
feration in absence of antibody was also determined
(O).

All six monoclonal anti-HBs, with varying
degrees of potency, enhanced proliferation of the
HBC-6 T cell clone. However, the enhancement caused
by the IgG antibodies was far more dramatic than
that caused by the IgM antibodies. The monoclonal
anti-TT did not affect HBsAg-induced proliferation
of the HBC-6 clone.

In another experiment, ten different mouse
monoclonal anti-HBs were tested for the ability to
enhance HBsAg-induced proliferation of a different
HBsAg-specific T cell clone, the HBC-4 clone.

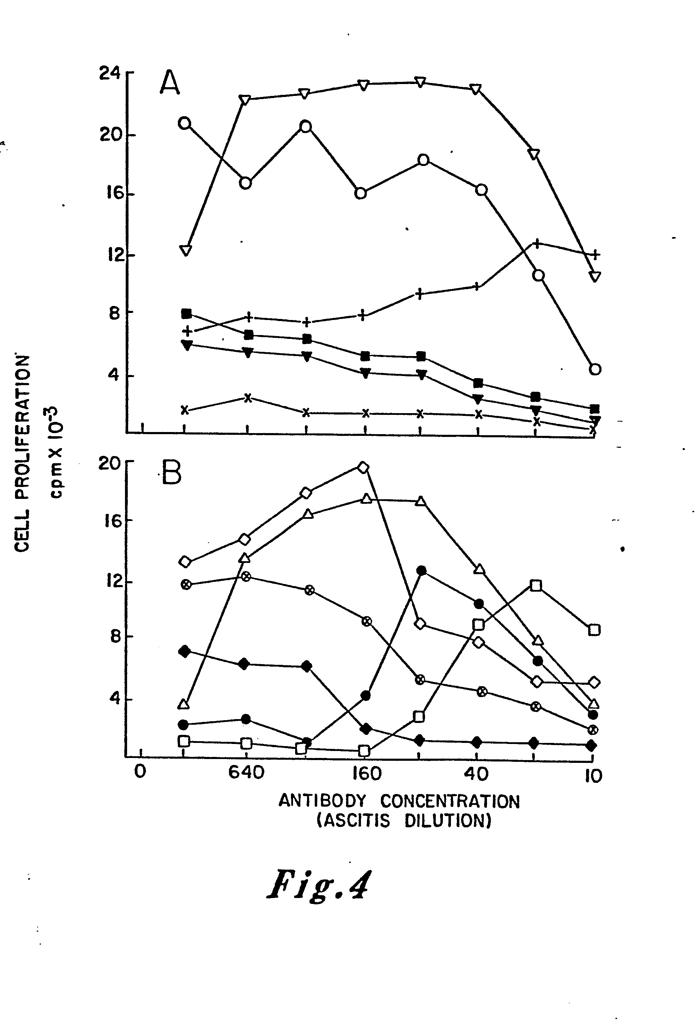

Antigen-induced proliferation was measured with
different concentrations of antibodies and a constant concentration of HBsAg (1 ug/ml) in a single
experiment. The results shown in Figure 4, are
separated into two panels for simplification. The
HBsAg preparation used in this experiment was the
same as in Figure 3. The following mouse monoclonal
anti-HBs were tested:
▽ , H2C4 (IgG$_1$); O , A2C6 (IgG$_1$); ◇ , H4B8 (IgG$_1$);
△ , A5C6 (IgG$_1$); ⊗ , H4G7 (IgG$_1$); ☐ , A5E6 (IgG$_{2b}$);
◈ , H1F8 (IgM); ◉ , Ay10 (IgM); ▣ , H2F11 (IgM);
▼ , H5D3 (IgM). Human affinity purified polyclonal
anti-HBs (+) were also used in this experiment

-38-

(undiluted concentration = 200 ug/ml). Controls were:

X , monoclonal anti-tetanus toxoid B2TT (IgG$_{2a}$);

✳ , no antibodies.

The IgG antibodies were generally more effective in enhancing HBsAg-induced proliferation of HBC-4 cells than the IgM antibodies (Figures 4A and 4B). The only exception was that the monoclonal antibody A5E6, the only IgG$_{2b}$ examined, was not as effective as the IgG$_1$ and IgG$_{2a}$ antibodies. It was about as equal or less effective than IgM antibodies. Additionally, most monoclonal IgG anti-HBs increased the HBsAg-induced proliferation of the T cells to a greater extent than did purified human polyclonal anti-HBs (Figure 4A). As with HBC-6, the mouse monoclonal anti-tetanus toxoid antibody had no effect on the antigen-induced proliferation of this T cell clone.

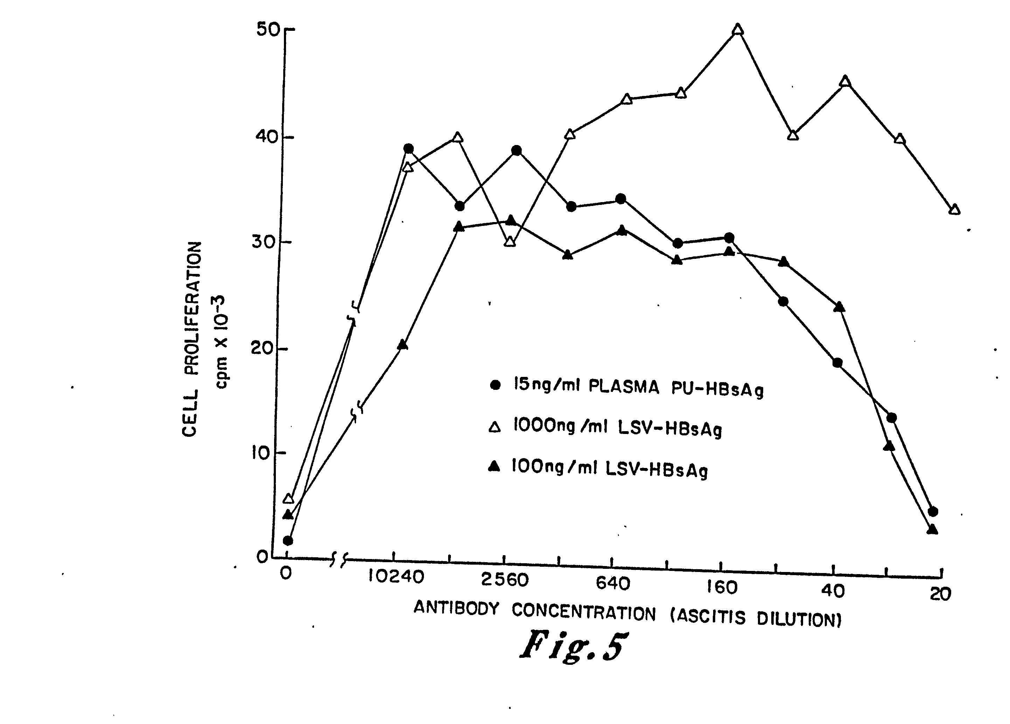

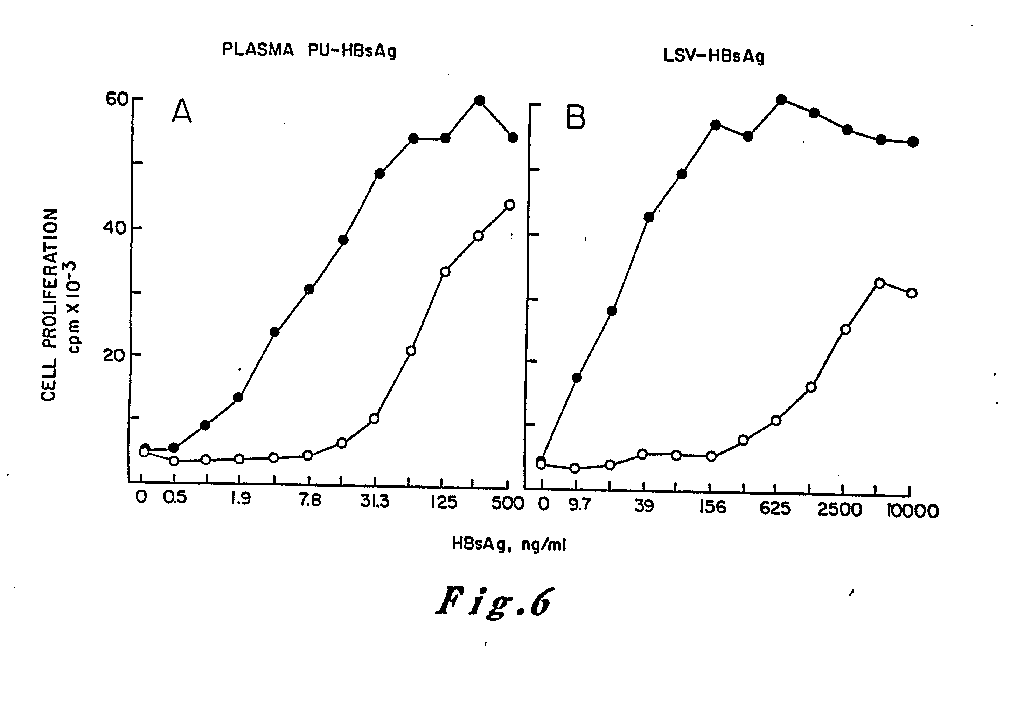

The potentiating effect of one of the (IgG$_1$) monoclonal anti-HBs on the antigen-induced proliferation of cloned T cells was also examined with two different preparatons of HBsAg, which do not contain human serum proteins. One preparation (PU-HBsAg) was also derived from plasma of chronic carriers but was treated with pepsin and urea to remove the complexed human serum proteins. The other HBsAg preparation (LSV-HBsAg) was purified from culture supernatants of a genetically engineered cell line that harbored and expressed DNA encoding for HBsAg. In one experiment, different concentrations of the mouse monoclonal anti-HBs A2C6 was tested for the capacity to enhance HBC-6 proliferation at a constant concentration of the HBsAg preparations (Figure 5). In another experiment, the proliferation of HBC-4 was measured at different concentrations of PU-HBsAg (Figure 6A) or LSU-HBsAg (Figure 6B) in the presence of A2C6 antibody at 1:100 ascites dilution (●) or in absence of antibody (○). The results presented in Figures 5 and 6 show that the monoclonal anti-HBs was effective in enhancing the proliferation of HBC-6 cells to each of the HBsAg preparations.

The monoclonal anti-HBs also enhanced HBsAg-induced production of Interferon-gamma by the HBsAg-specific T cells. As shown in Table III, significantly more interferon-gamma was secreted by T cells when both anti-HBs and HBsAg were present together in the culture, than when HBsAg alone was present. In the absence of antigen, anti-HBs increased the production of interferon-gamma slightly.

-40-

## Table III

### The effects of a mouse anti-HBsAg monoclonal antibody on the HBsAg-induced production of IFN-$\gamma$ by a T cell line and clones

|  | IFN- produced, U/ml[a] | | | | | |
|---|---|---|---|---|---|---|
|  | PU-HBsAg[b] | | LSV-HBsAg[c] | | Medium | |
| T cells: | -Ab | +Ab[d] | -Ab | +Ab | -Ab | +Ab |
| HBC-1 | 2.5 | 9.17 | 0.66 | 9.79 | 0.23 | 0.30 |
| HBC-4 | 14.83 | 55.59 | 2.38 | 39.35 | 0.24 | 0.58 |
| HBC-6 | 2.99 | 13.13 | 0.20 | 9.00 | 0.09 | 0.16 |
| HBL | 18.87 | 69.00 | 40.50 | 113.74 | 5.59 | 13.56 |

[a] NIH Units/ml, measured as described in Example 1.

[b] 1 ug/ml of pepsin-urea treated HBsAg from human plasma.

[c] 1 ug/ml of HBsAg produced by recombinant DNA technology.

[d] 1:1000 ascites dilution of monoclonal IG1 anti-HBs "C61".

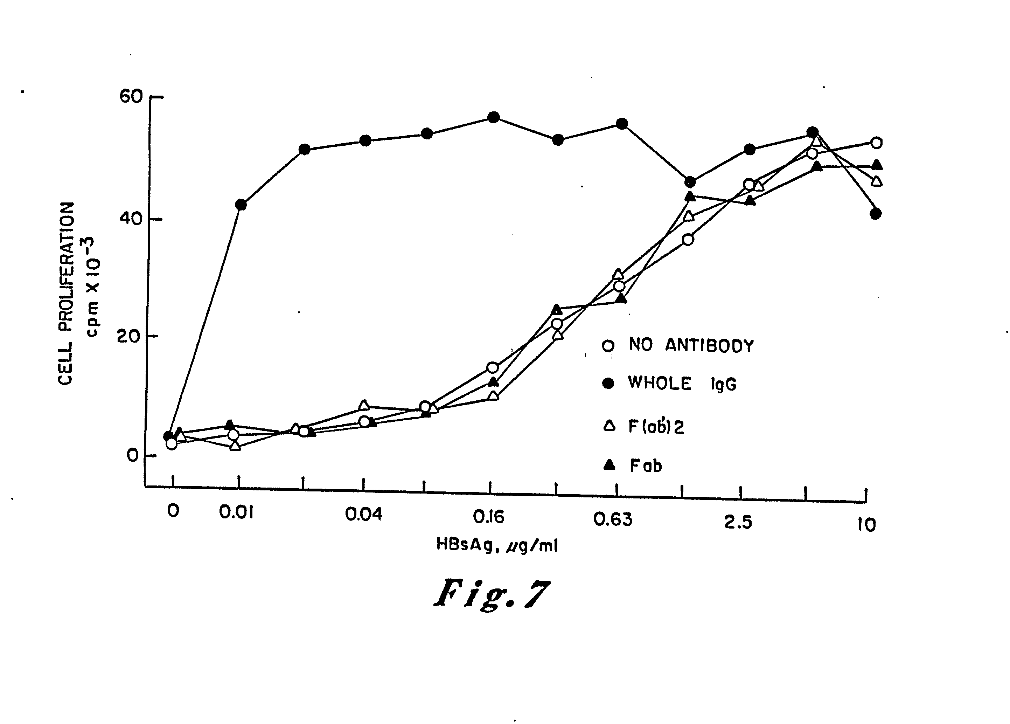

The Fc portion of the antibody molecule appears to be necessary enhancement of antigen-induced proliferation. Only intact anti-HBs molecules were capable of potentiating the HBsAg-induced proliferation of T cells. Figure 7 shows the reactivity of clone HBC-6 to different concentrations of plasma-derived HBsAg measured in the presence of a constant

-41-

amount (1 ug/ml) of the purified monoclonal anti-HBs A5C3; whole IgG molecules ( ● ), $F(ab')_2$ fragments ( △ )or Fab fragments ( ▲ ). Proliferation in the absence of antibody is also shown ( ○ ). Both $F(ab')_2$ and Fab fragments had no effect on proliferation of HBC-6 cells at any HBsAg concentration.

Equivalents

Those skilled in the art will recognize, or be able to ascertain, using no more than routine experimentation, numerous equivalents to the specific substances and procedures described herein. Such equivalents are considered to be within the scope of this invention and are covered by the following claims.

CLAIMS:

1. A method of enhancing an immune response of T lymphocytes to antigen _in vitro_, comprising immunizing said lymphocytes with a complex of the antigen and antibody specific for the antigen, said complex formed with a molar excess of antibody.

2. A method of claim 1, wherein the antibody is a monoclonal antibody, e.g. of the $IgG_1$ or $IgG_{2a}$ subclass.

3. A method of potentiating antigen-induced proliferation and effector function of T lymphocytes maintained in culture comprising administering to said culture a complex formed of the antigen and a molar excess of monoclonal $IgG_1$ or $IgG_{2a}$ antibody specific for the antigen.

4. A method of isolating antigen-specific T lymphocyte clones, comprising the steps of:
   a) immunizing cultured lymphocytes with a complex of the antigen and an antibody specific therefor, said complex formed with a molar excess of antibody, to activate and expand the population of antigen-specific T lymphocytes;
   b) cloning the antigen-activated lymphocytes; and
   c) selecting a T lymphocyte clone specifically reactive to the antigen.

5. A method of producing B lymphocyte clones which produce antibody specific for an antigen, comprising the steps of:
   a) immunizing cultured lymphocytes with a complex of the antigen and an antibody specific for the antigen, said complex formed with a molar excess of antibody, in order to expand the population of B lymphocytes which produce antibody against the antigen;

b)    cloning the antigen-activated B lymphocytes;  and

c)    selecting a lymphocyte clone B which produces antibody specific for the antigen.

6.    A method of producing hybridoma cells which produce antibody specific for an antigen, comprising the steps of:

a)    immunizing cultured lymphocytes with a complex of the antigen and an antibody specific for the antigen, said complex formed with a molar excess of antibody, in order to activate and expand the population of B lymphocytes which produce antibody against the antigen;

b)    fusing the antigen-activated B lymphocytes with neoplastic lymphoid cells to form hybridomas;

c)    cloning the hybridoma cells;  and

d)    selecting a hybridoma clone which produces antibody specific for the antigen.

7.    A method of enhancing immunogenicity of an antigen, comprising forming a complex of the antigen and a monoclonal $IgG_1$ and $IgG_{2a}$ antibody specific therefor, said complex formed with a molar excess of the antibody.

8.    A method of enhancing an immune response in vivo to an antigen comprising administering a complex of the antigen and a monoclonal antibody of the $IgG_1$ or $IgG_{2a}$ class specific for the antigen, said complex formed with a molar excess of antibody.

9.    A method of claim 1 or claim 8, wherein the antigen is a viral, bacterial or tumor-associated antigen, or portion thereof.

10.    A method of enhancing immune response to an antigen in vivo, comprising administering a complex of antigen coupled to a carrier, (e.g. a protein or carbohydrate carrier) and a monoclonal antibody of the $IgG_1$ or $IgG_{2a}$ subclass specific for the carrier, said complex formed with a molar excess of antibody.

11.    An immunogen composition comprising a complex of antigen and monoclonal $IgG_1$ or $IgG_{2a}$ antibody specific therefor in a physiologically acceptable vehicle.

12.    An immunogen composition comprising a complex of antigen coupled to a carrier (e.g. a protein or carbohydrate carrier) and monoclonal $IgG_1$ and $IgG_{2a}$ antibody specific for the carrier in a physiologically acceptable vehicle.

13.    An immunogen composition of claim 11 or claim 12, wherein the antigen is a viral, bacterial, or tumor-associated antigen, or a portion thereof, a synthetic antigen or antigen produced by recombinant cells.

14.    A method of enhancing an immune response of T lymphocytes to Hepatitis B surface antigen (which is e.g. soluble or particulate) in vitro, comprising contacting said lymphocytes with a complex of Hepatitis B surface antigen, said complex formed with a molar excess of antibody.

15.    A method of claim 4 or claim 14, wherein the antibody is a monoclonal antibody of the $IgG_1$ or $IgG_{2a}$ subclass.

16.    A method of enhancing an immune response to Hepatitis B surface antigen comprising administering a complex of Hepatitis B surface antigen and a monoclonal antibody of the $IgG_1$ or $IgG_{2a}$ subclass specific for Hepatitis B surface antigen.

17.    A vaccine composition for vaccination against Hepatitis B virus, comprising a complex of Hepatitis B surface antigen and monoclonal antibody of the $IgG_1$ or $IgG_{2a}$ subclass specific for Hepatitis B surface antigen.

Fig.1

0153871

Fig.2

Fig.3

0153871

*Fig.4*

Fig.5

0153871.

Fig.6

Fig. 7